# EUROPEAN PATENT SPECIFICATION

(11) **EP 0 971 737 B1**
(45) Date of publication and mention of the grant of the patent: **11.05.2005**
(21) Application number: 98907815.9
(22) Date of filing: 05.03.1998
(51) Int. Cl.: A61K 39/39, A61K 39/29, A61K 39/295

(54) **IMMUNOPOTENTIATING FORMULATIONS FOR VACCINAL USE**
ZUSAMMENSETZUNGEN ZUR IMMUNVERSTÄRKUNG VON IMPFSTOFFEN
FORMULATIONS IMMUNOSTIMULANTES A USAGE VACCINAL

(30) Priority: 06.03.1997 CU 2797
(43) Date of publication of application: 19.01.2000
(73) Proprietor: CENTRO DE INGENIERIA GENETICA Y BIOTECNOLOGIA (CIGB), Ciudad de la Habana 10600 (CU)
(72) Inventor: AGUILAR RUBIDO, Julio César, Ciudad de la Habana 32200 (CU); MUZIO GONZALEZ, Verena Lucila, Ciudad de la Habana 10600 (CU); LEAL ANGULO, Maria de Jesús, Ciudad de la Habana 12100 (CU); GUILLEN NIETO, Gerardo Enrique, Ciudad de la Habana 10400 (CU); PENTON ARIAS, Eduardo, Ciudad de la Habana 12100 (CU); VELIZ RIOS, Gloria, Ciudad de la Habana 11500 (CU); PICHARDO DIAZ, Dagmara, Ciudad de la Habana 13600 (CU); HERRERA BUCH, Antonieta, Ciudad de la Habana 12100 (CU); IGLESIAS PEREZ, Enrique, Habana del E., Ciudad de la Habana 11700 (CU); CRUZ RICONDO, Luis Javier, Ciudad de la Habana 11000 (CU); CARMENATE PORTILLA, Tania, Ciudad de la Habana 10900 (CU); MESA PARDILLO, Cirse, Guanabacoa, Ciudad de la Habana 11100 (CU); HECHAVARRIA GAY, Maydel, Lawton, Ciudad de la Habana 10700 (CU); DIAZ MARTINEZ, Maylin, Ciudad de la Habana 10600 (CU); MADRAZO PINOL, Juan Joel, Playa, Ciudad de la Habana 12100 (CU)
(74) Representative: Prins, Adrianus Willem, Mr. Ir.
(86) International application number: PCT/CU1998/000003
(87) International publication number: WO 1998/039032

(56) References cited:
- EP-A- 0 418 626
- EP-A- 0 619 117
- WO-A-93/14195
- WO-A-93/24148
- DE-A- 19 627 392
- I. IDÄNPÄÄN-HEIKKILÄ ET AL.: "The antibody response to a prototype liposome vaccine containing Neisseria meningitidis outer membrane protein P1 produced in Bacillus subtilis" VACCINE, vol. 13, no. 16, 1995, pages 1501-1508, XP004057407
- DATABASE WPI Week 9638 Derwent Publications Ltd., London, GB; AN 96-379257 XP002066843 & JP 08 183 742 A (DAIICHI PHARM. CO. LTD.) , 16 July 1996
- SCHIRMBECK ET AL: 'Antibody and cytotoxic T-cell responses to soluble Hepatitis B virus (HBV) S antigen in mice: Implication for the pathogenesis of HBV-induced hepatitis.' JOURNAL OF VIROLOGY vol. 68, May 1994, pages 1418 - 1425

## Description

### Technical branch

The present invention is related to the field of medicine, particularly to the development of new immunological potentiation formulations allowing the increase of the amount and quality of the immune response to vaccine antigens.

The technical aim of the proposed invention is the development of formulations that are capable of increasing and/or modulating the levels of the immune response of the body to vaccine antigens.

### Previous technique

The adjuvants are substances that increase or optimize the immune response to antigens inoculated through the mucosal or systemic routes. The adjuvants or their formulations are combined with the antigen to generate or potentiate the type of response desired, decrease the number of inoculations and reduce the amount of antigen needed to obtain and maintain protection (McElrath, M.C. 1995 Seminars in Cancer Biology 6: 375-385).

Adjuvants have been developed through the need posed by the advancement of modern biotechnology with the production of pure soluble recombinant and synthetic antigens. In general, these antigens are safe, but of a decrease immunogenicity compared to those of the original organism. An important function of adjuvants or their formulations, is to increase the complexity of the antigen facing the immune system, in a safe way, thus increasing its immunogenicity (Alving, R.C. 1992 AIDS Res. Hum. Retroviruses 8 (8):1427-1430).

At present, the search for new adjuvants and immunological stimulators, as well as the development of new ways of delivering antigens and pharmaceuticals, is one of the prime lines of world research in the pharmaceutical field, especially in vaccines. The development of the adjuvants for mucosal use is a present need in the vaccine field (Report of the Expert Panel VI: Concerted efforts in the field of mucosal immunology 1996 Vaccine 14: 644-664).

The adjuvants may be -classified as mucosal and systemic adjuvants, considering that the physiological characteristics on receiving and processing the antigen in both inoculation routes, generate different procedures of adjuvation. The mucosal route, according to the characteristics of the antigen, require binding or coating procedures with specific ligands that send the antigens to the M cells. The adjuvant activity for mucosal antigens is obtained through strategies that help the antigen to cross the borders imposed by the route. The physical characteristics of the antigen may favor its phagocytosis.

Once the antigen has been assimilated, the adjuvant may influence the response by any of the known mechanisms: the adsorption of the antigen, the deposition effect, the cytokine induction, the activation of the complement the recruiting of different cell populations of the immunological system, the delivery of antigen to different antigen presenting cells, the regulation of the expression through class I or class II and the stimulation of the production of different subtypes of antibodies (McElrath M.C. 1995 Seminars in Cancer Biology 6: 375-385).

Some of the immunological stimulators known as the muramyl-dipeptide (MDP), monophosphoryl-lipid A (MPL), the lipoid amine Avridine and those known as toxins of *V*. *cholerae* (CT) and of *E. coli* (HLT), are recognized adjuvants for antigens administered through the mucous route (Walker, R.I. 1994 Vaccine 12 (5): 387-400).

The MDP and MPL have been studied in liposomal formulations for therapeutic and prophylactic use, however, the toxins and their sub-units (specially the CT and CTE) are the most common mucosal adjuvants.

The ability of CT to act as an oral adjuvant has been confirmed by a large number of researchers (McGhee, J.R. et al. 1992 Vaccine 10 (2): 75-88). The cholera toxin does not fulfill the classical definition of an adjuvant because it stimulates an immune response against itself and its adjuvant activity depends on its immunogenicity (Elson, C.O. 1987 Fed. Proc. 46: 1778). The immunomodulating effects of the CT and HLT explaining their strong adjuvant activity, include the increse of the presentation of the antigen by several types of B cells, the increase in the differentiation of B cells to the IgA isotype, the interaction with T cells and the production of cytokines (Lintermans, P. 1995 Advanced Drug Delivery Reviews 18: 73-89).

From the practical point of view, the use of the holotoxin in man is not possible due to its toxicity. A better strategie is the detoxification of the CT, by separating the sub-unit A or through mutations of the gene codifying it. CT as well as the CTB (non-toxic sub-unit) may potentiate the immune response to several antigens bound covalently due to specific interactions with M cells (Holmgren, J. et al. 1993 Vaccine 11: 1179-1184).

The antigen delivery systems have reached a sufficiently high degree of development as to have an impact on immunization. It is expected that the solid particulated systems for parenteral or non-parenteral administration should be among the first licensed products (Li Wan Po et al. 1995 Advanced Drug Delivery Reviews 18: 101-109).

Through the possibilities offered by the antigen delivery systems in the particulation of soluble antigens, and taking advantage of the physiological characteristics of the mucosal route, these systems have been tested and have shown adjuvant activity. In the literature they have been classified as:
a) synthetic/inactivated and b) alive (Report of the Expert Panel VII: Vaccine Delivery Systems 1996 Vaccine 14: 644-664).

In respect to the first group, the artificial polymeric particles have been studied with different results comprising: the co-polymeric microspheres of lactic and glycolic acids, also alternative polymers as polyphosphacenes, cellulose acetate polymers, iminocarbonates, ethylenvinyl acetate polymers, proteinoid microspheres, dextran polyanhydrid and nanospheres; the particles produced from natural materials: alginates, gelatins and seeds of plants and also the liposomes and their variants: proteoliposomes, virosomes and ISCOMs (Li Wan Po et al. 1995 Advanced Drug Delivery Reviews 18: 101-109).

The size of the particles is within the group of important factors for antigen sending. In the case of the mucosal immunization route it has been reported that particles of a diameter greater than 10µm are not absorbed (Eldridge J.H. 1990 J. Control. Release 11:205-214). In experiments with rats it was observed that after oral administration, only particles of 5µm deeply penetrated the Peyer plaques and those of 1µm of diameter, penetrated the lymphonodes and the liver and went into the bloodstream (Jani, P. et al 1990 J. Pharm. Pharmacol. 42:821-826)(Alpar, H.O. et al. 1989 J. Pharm. Pharmacol 41:194-196).

The extrapolation of these results in man is not yet defined and sometimes the adsorption by the gastrointestinal tract is not a requirement for adjuvant activity although it has been proven that with the adsorption of the diphtheria toxoid in plant seed of up to 2mm diameter, the immune response was potentiated (Mirchamski, H. et al. 1994 Vaccine 12:1167-1172).

The determination of the optimum size of the controlled release systems through the oral, rectal or vaginal routes is under study (Li Wan Po et al. 1995 Advanced Drug Delivery Reviews 18:101-109).

Another factor affecting the particles is the hydrophobic-hydrophilic balance, which may be modified to obtain a modulation of the immune response (Jani, P. et al 1990 J. Pharm. Pharmacol. 42:821-826).

Recently, the use of vaccines of protein cocleates described in 1975 has been patented (Papahadjopoulos D. et al 1975 Biochem. Biophys. Acta 394: 483).

The cocleates are complexes of liposomes and divalent cations (mainly calcium) that, through the calcium-fosfolipid interaction, enable the formation of a liposome structure coiled on itself, allowing the immunization through different routes (Gould Foguerite, S. WO 95/09648). With this structure, the immune response of antibodies, as well as the cell mediated responses are stimulated (Gould Foguerite, S. 1994 AIDS Res. Hum. Retroviruses 10 (Supl. 2): S99-S103).

The absorption and passage of the antigens through specialized cells of the epithelium associated to the lymphoid follicle, the M cells (Microfold) is a critical step in the generation of an immune response through the simple intestinal epithelium, which is generally accepted by all authors. Through this process the antigens are sent to the basal pocket of the M cells, constituting the first contact of the antigen (practically undegraded) with B and T cells and macrophages. The main function of the pocket is of providing the organism with an environment protected from the modulator influence of the external humoral factors (Neutra, R.M. 1996 Annu. Rev. Immunol. 14: 275-300).

In the respiratory tract, the epithelium varies from pseudostratified, to simple. In the bronchi, the simple epithelial zone, the intercellular spaces are sealed by tight binds and the main mechanism for the antigens to enter is through the M cells. At the tonsils, the predominant epithelium is the stratified epithelium. Here, the absorption mechanism of the antigen is closely associated to a net of macrophages and mobile dendritical cells from the bone marrow, of up to 700 cells per mm². These cells are able to migrate to the organized lymphoide tissue associated to the mucous (O-MALT) or to a lymphonode, presenting the processed antigen that was phagocyted at the surface of the tonsils. Under normal conditions this constitutes the main mechanism for the presentation of the antigens through MHC class II of the respiratory tract (Neutra, R.M. 1996 Annu. Rev. Immunol. 14: 275-300).

The difference in the absorption of the antigen at the oral mucous and nasal-pharyngeal levels allow us to understand why the inoculation of an adjuvant through both routes will not necessarily produce the same results. Therefore, it is not obvious that an adjuvant that is effective through the oral route, will or will not be effective by the nasal-pharyngeal route. The adjuvants may have different effects in different mucous sites, since different mucous surfaces have different micro-environments. The progress in the knowledge of the physiology of the mucous and the mode of action of the adjuvants may help the development of more effective mucosal vaccines, as well as the characteristics of the antigen as: size, presence of mucosal ligands, electric charge, lipophilicity and T dependence may also affect the immune response. (Report of the Expert Panel VI: Concerted efforts in the field of mucosal immunology 1996 Vaccine 14: 644-664).

At the same time, other elements of the organism as the hormonal fluctuations during the menstrual cycle, affect the assimilation of the antigen at the vagina. This fact shows the strong collaboration between the dendritic and epithelial cells and explains the variation in the effectiveness of the vaccines through the vaginal route (Parr, E.L.,Parr, M.B. 1992 Vaccine Res. 1: 221-25).

For the case of the simple epithelial route of the intestine, the delivery of non-living vaccines to the M cells has been difficult, given the fact that the unprotected macro-molecules are readily digested or dragged by the secretions and the motility of the gastrointestinal tract. There is little information available in relation to the components of the apical membrane of the M cells that may serve as receptors. The liposomes and the micro-particles may adhere to the mucous surfaces by hydrophobic interactions, but the entrance of the antigens to the M cells is inefficient since they are rapidly trapped in the mucous gels and many do not get to reach the mucous. The macro-molecules or particles that conjugate or coat with ligands as the CTB, have the limiting factor of their access to the receptors (Neutra, R.M. 1996 Cell 86: 345-348).

Recent experiments in mice, using 28.8 nm coloidal gold particles coated with CTB showed that they were able to adhere and penetrate selectively in M cells of the epithelium associated to the mucous follicle, being unable to penetrate the enterocytes of the so-called "brush border". Larger particles; of approximately 1.13 µm; also coated with CTB, were unable to adhere to M cells, while CTB-FITC particles of approximately 6.4 nm, adhered and entered through both cell types. This experiment demonstrated the role of the glyco-chalix in the adherence and entering of the antigens through the M cells. The B subunit of the cholera toxin has receptors both in the enterocytes as in the M cells. The use of the ligands bound to particles may result in a specific adherence to M cells, but only in a range of sizes restricted by the glyco-chalice. Particles of 1µm or greater, require ligands that are specifically directed to components of the M cells. The identification of these components is still under study (Frei, A. et.al. 1996 J. Exp. Med. 184: 1045-1059).

A group of pathogenic bacteria is able to surpass the difficulties of the non-living systems on being efficiently assimilated by the lack of receptors at the M cells. These bacteria exploit this mechanism to infect mucous tissues and disseminate themselves systemically before being detained by the immune system. The bacterial pathogens that adhere to the surface of the M cells start the signal transduction events at the epithelial level that promote their entrance. The best known one is that of attenuated bacterial strains of *S. typhi* ty21a, for which a lectin type interaction with the receptors of a polysaccharide nature of the cell membrane of M cells. Also safe and effective are the attenuated live strains of *V*. *cholerae* and polio virus for oral immunization. The genetically manipulated strains of these microorganisms were first used in man as carriers of heterologous antigens (Mekalanos, J.J. 1992 Adv. Exper. Med. Biol. New York Plenum Press: 43-50).

The biology of these live vectors introduce new challenges. The vaccine strains of *V*. *cholerae*, that have no genes for the toxin, may still produce diarrhea, seemingly because the epithelial cells release cytokines as a response to bacterial adherence (Mekalanos, J.J. 1992 Adv. Exper. Med. Biol. New York Plenum Press: 43-50). The main challenge in the use of the genetically manipulated strains of the *S. typhi* and *S. typhimurium* consist in obtaining a sufficient attenuation offering safety while still adhering to the M cells and their proliferation in the mucous, to maintain their immunicity. The attenuated strains of *Shigella*, are also internalized by the M cells, but they have lost their ability to disseminate from cell to cell. This phenomenon is the basis of the attenuation, but there is still the release of local cytokines and chemotactic factors that may cause the rupture of the normal function of the epithelial barrier (Sansonetti, P.J. 1991 Rev. Infect. Dis. 13: 285-292).

The virus are also found in this study. The fact that the polio-virus type 1 and the attenuated strain of Sabin use the transportation through the M cells to cross the epithelial barrier, make them important candidates for oral vaccines to send foreign antigens in man. The vaccinia and other pox virus are assimilated by the mucous surface but their interaction with the M cells is still unknown (Report of the Expert Panel VI: Concerted efforts in the field of mucosal immunology 1996 Vaccine 14: 644-664).

Many complex carbohydrates of natural origin stimulate the cells of the immune system and the reticulum-endothelium system (Davis, S.E. 1975 Am. Chem. Soc. Sympos. Series 15, Jeanes A. Hodge J. Eds. Am. Chem. Soc. Washington D.C.). Among these are the polymers of plants and funguses as the glucans, dextrans and lentinans, all of which are glucose polymers, and the mannans, among which are found the glucomannans and the galactomannans.

Also found are the levans and xylans (Tizard, I.R. et al. 1989 Mol. Biother 1:290-296). The activity of many of these polyglycans on the macrophages (having glucan and mannan receptors) include the induction of phagocytosis and the secretion of cytokines, leucotriens and prostaglandines. The lentinan, a glucan that is common in mushrooms, stimulates the cell and antibody response in sheep eythrocytes while levan is mytogenic for B cells and a macrophage activator (Simon, P.M. 1994 Exp. Opin. Invest. Drugs 3 (3):223-239).

The acemannan is a mannan composed of mannose with O acetylations in approximately 8 out of every 10 remains. It is extracted as a major component of the mucilagous substance or gel of the leaf of *Aloe barbadensis* Miller, medicinal plant used throughout history. Different tests in vitro indicate that the manans activate the monocyts and macrophages inducing the production of interferon-γ, factor-α of tumoral necrosis, colony stimulator factor of monocytes and granulocytes, IL-1β and IL-6 (Peng, S.Y. et al. 1991 Mol.Biother. 3: 79-87). The acemannan potentiates the generation of cytotoxic T lymphocytes (CTL) (Womble, D. et al. 1988 Int. J. Immuno-pharmacol. 10:967-974), the cytotoxic activity of Natural Killer (NK) cells (Marshall G.D. et al. 1993 J. Immunol. (part II) 150: Abstr 1381), and also, slightly, the *in vitro* human alloresponse.

The increase of the cytotoxic activity and the secretion of γ interferon supports the antiviral and antitumoral therapeutic use of acemannan. Its antiretroviral activity was evidenced in animals in the case of feline leukemia (Sheets, M.A. et al. 1991 Mol. Biother. 3: 41-45). Clinical assays in AIDS and cancer patients are currently in course.

Patents have recently been applied for in relation to the use of the Acemanan as an adjuvant for vaccines. (McAnalley, B.H. EP 0 619 117 A2, Nordgrem, R.M. WO 93/14195), but in none of the two cases the naso-pharyngeal use of the acemannan is protected. In both patents the antigens are inoculated by the systemic route (subcutaneous and intramuscular). In relation to the mucosal route, the first patent shows poor results in the oral use of a acemannan formulation. The second patent widens the use to eye drops. The results obtained with an acemannan oral formulation (shown in the first patent) may be considered poor compared to those obtained by the systemic inoculation route. As previously explained, the adjuvants may have different effects in the different mucous sites due to the physiology of the assimilation of the antigen and the different environment in each route, that may affect the activity of the immunological potentiator (Report of the Expert Panel VI: Concerted efforts in the field of mucosal immunology 1996 Vaccine 14: 644-664).

Besides the differences between the mucosal routes, different results have also been obtained when the same adjuvant is used systemically or through the mucous. This is the case of the most common systemic adjuvant used, the aluminum hydroxide. This adjuvant has not been more effective than the PBS when mice are inoculated through the oral and nasal routes with antigens for which it is the traditional adjuvant for systemic use - as for example the tetanus toxoid - (Alpar H.O. et al. 1992 Int. J. of Pharm. 88: 335-344). Therefore, it is not obvious that a systemic adjuvant is also necessarily a mucosal adjuvant.

### Detailled description of the invention

In the present invention we report for the first time a vaccine formulation of nasopharyngeal administration, having as its main components the surface antigen of the Hepatitis B virus (HBsAg) and the acemannan, in adequate proportions.

This formulation is a novelty due to the properties resulting from its mucosal administration, these are: to generate a systemic immunity of similar intensity and higher quality for similar antigen doses to that obtained with conventional vaccine formulations using aluminum hydroxide as the adjuvant. Also, a strong response at the mucous is generated, which is not obtained by systemic inoculations of the HBsAg.

We also demonstrated that with the systemic use of the formulation of HBsAg with acemannan, the immune response was significantly increased both quantitative and qualitatively compared to conventional vaccine formulations using aluminum hydroxide as the adjuvant.

This is the first time that the use of the HBsAg is reported as antigen delivery system through the nasopharyngeal route combined with non-specific immunity activator polysaccharides. With this system a proportionally increased response was obtained for the epitopes displayed on its surface in relation to the proportion obtained with other adjuvants. This finding allows the formulation of combined vaccines using the HBsAg as an antigen delivery system through the mucous. It also supports the use of this strategy for the soluble antigen-particulated antigen combination and its use with polysaccharides with these characteristics through mucosal inoculation routes.

Here, it was generalized that liquid formulations of particulated antigen delivery systems together with the acemannan inoculated by the nasopharyngeal route preferentially potentiated the immune response in relation to the soluble antigen until the level of serological response obtained by the systemic route with other adjuvants was surpassed. The increase in the quality of the response to enhance the level of antibodies of subclass IgG2a in Balb/C mice, is demonstrated. This aspect constitutes a qualitative superiority in relation to the aluminum hydroxide, enabling the design of vaccines that are antitumoral and against microorganisms in the cases where the induction of the Th1 responses are required. It has recently been observed that the cytokines produced by the Th 2 cells are those associated to the mucosal immunity and that the T cells in the mucous lymph nodes are more liable to produce type Th2 cytokines (Meeusen, E.N.T. 1996 Immunology Today 17 (9): 421-424) which confirms the value of this finding.

In the present invention the immunological modulator activity of combined formulations of antigen-acemannan are also described. We report for the first time the specific potentiator activity of the Th 1 response in relation to other commonly used adjuvants, through the nasopharyngeal and systemic routes for particulated and soluble antigens. For the latter, the humoral response is of similar intensity to that obtained with the aluminum hydroxide, but it is qualitatively different, hence the immunological modulator effect reported.

These results support its introduction in vaccine formulations for immunoprophylaxis and immunotherapy of diseases caused by intracellular pathogens and cancer.

At present the action mechanisms of the acemannan and other immunological stimulator polysaccharides is not completely clear. A proposed mechanism deals with the macrophages and dendritic cells, that have specific receptors for antigen standards present in the surface of the pathogens discriminating those that are dangerous and those that are not. According to this, at the site of the systemic inoculation there is a strong monocytemia. The antigenic assimilation at the nasopharyngeal level and more specifically at the Waldeyer ring - according to the assimilation mechanism for stratified epithelial tissue- can be potentiated with the activation of the macrophages and dendritic cells found in the area and with the attraction toward this zone of an increased number of immunological competent cells.

An important proportion of the dry weight of the gel blade of the Aloe plant is constituted by Calcium Oxalate crystals (Carpenter, H.R. P.N. 5 118 673). These have been the object of recent studies due to the major presence in kidney stones, which is a common pathology (Lieske, J.C. et. al. 1994 Proc. Natl. Acad. Sci. U.S.A. 19; 91(15): 6987-91). Sinice 1993, certain macromolecules have been described that may be adsorbed by the crystal (Stapleton, A.M. et. al. 1993 Kidney Int. 44(4):817-24). More recently, other proteins have been found: Nephrocalcin, Tamm-Horsfall protein, Uropontin and Renal Lytostatin (Berland, Y. et al. 1993 Nephrologie 14(4):183-7).

Experiments carried out by our group demonstrated the adsorption of the HBsAg to the calcium oxalate crystal as well as the co-precipitation of the DNA with that salt, obtaining reduced size particles due to the characteristics of the salt and the processes of inhibition of the agglutination of the crystals that occur in a polysaccharide rich medium as is the acemannan.

For the systemic inoculation of the HBsAg, the combination of the components of the Aloe gel also generated better results to those obtained with the separate elements.

The particulation of the antigens, through colloidal salts as well as conjugations to particulated antigens or their inclusion or association to delivery systems of antigens, constitutes a first step in antigen processing in this invention, the second step is the addition of the polysaccharide that activates the immune system.

The viscous consistency of the acemannan makes it an active vehicle that increases the time the antigenic particle remains at the inoculation site. Other activities as the induction of the cytokines, the activation of mechanisms for the uptake of antigens by the M cell, the recruitment of different populations of cells from the immunological system and the increase of the antigen presenting activity, are not discarded.

The formulation which is the object of this invention presents, according to the size of the species to be immunized, an amount of inoculum able to cover the nasopharyngeal area up to the subglotis. The dose may be divided in 2 parts for its application or it may be introduced once. The concentration of the polysaccharide that guarantees an optimum immunologic response and at the same time an adequate viscosity for a better antigen retention in the mucous, is in the range of 1mg/mL to 12mg/mL. Within the requirements of the antigens are its particulated character since it has been proven that this nasal formulation is not equally effective with the same soluble antigen or without the ligand effect which is specifically found in the mucous. Therefore, the positive result with a certain type of antigen does not guarantee a good result for another type, for which reason the characteristics of the antigen of being particulated or having ligand activity with epithelial cells constitutes a strong requirement of the antigen, which is attained with the particulated methods presented.

The formulations that are the objective of this invention allow to obtain high levels of seric antibodies and a more complete response in relation to immunoglobulin isotypes and subclasses, that are greater in amount and quality to those obtained when inoculating the soluble antigen or using another adjuvant.

The immune responses obtained serologically through the mucosal administration of these formulations is comparable to those obtained with systemic inoculations using conventional adjuvants, but it is much higher at the mucous level.

The low reactogenicity at the mucous in relation to adjuvants of the toxin type and attenuated microorganisms, together with a lower cost, the independent T response for the adjuvant and its modulator activity towards a Th1 response, allows to generate higher quality responses. On attaining a greater immunogenicity of the antigens, the number of inoculations may be reduced as well as the amount of antigen per dose.

### EXAMPLES OF PERFORMANCE

### Example 1:

The acemannan polysaccharide is obtained from the total aquous extract of the *Aloe barbadensis* Miller leaf. Leaves are collected form plant of 2 to 3 years of age and are stored for 2 to 3 days at 4°C in a dark room. Later the end and borders of the leaves are eliminated. The leaves are divided in small portions and ground while occasionally adding a minimum amount of sterile water to facilitate the process. The mucilage (or gel found within the leaf) was also used to obtain the acemannan.

The leaves or the gel, after grinding, were centrifuged at 10 000 rpm, eliminating all the insoluble remains that were mainly from the epidermal and fibrous tissues of the leaf. The lyophilized supernatant was named total extract (Te). The Te was resuspended according to convenience for a later 80% ethanol precipitation with slow stirring. Precipitation temperature was of 4°C. After 24 hours the solution was centrifuged for 20 min at 10 000 rpm and the supernatant was discarded. The precipitate was resuspended in sterile distilled water and lyophilized. This product is called ethanol precipitate.

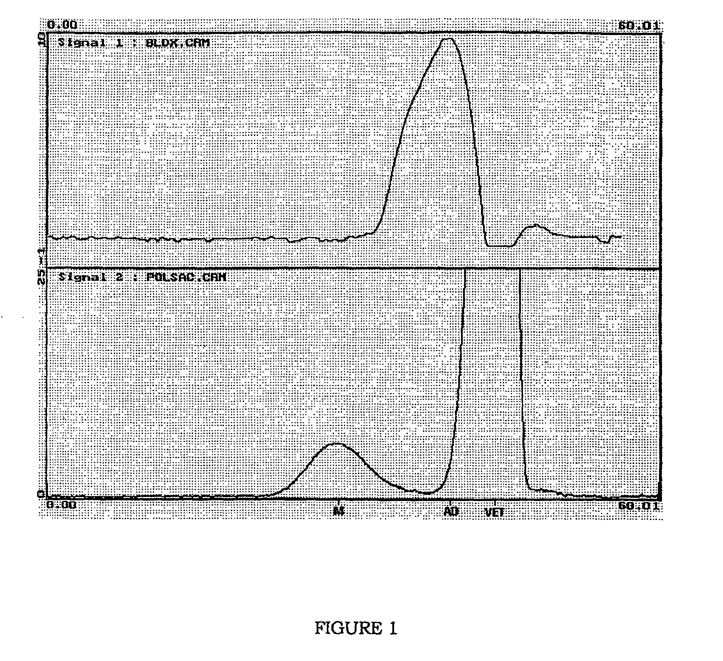

To obtain the acemannan with a high degree of purity, molecular exclusion chromatrography in Sepharose CL-4B was chosen. A phosphate buffer saline solution (PBS) or 0.2 M NaCl was used for the run, as well as for resuspending the sample. Figure 1 shows the chromatogram of a HPLC chromatography in a filtration gel column TSK G6000PW before the gel filtration in Sepharose CL-4B. In this chromatogram the point corresponding to the elution volume of the Dextran Blue (DB) of MW=2000 kDa is pointed out, and at the end is a point corresponding to the total elution volume (TEV). The first peak (M), of a higher molecular weight than the DB was positive for the Antrone colorimetric method (Trevelyan W.E. y Harrison J.S. 1952 Biochem J. 23: 1824).

The analysis through infra-red spectroscopy of both peaks showed the presence of bands that are characteristic of the acemannan at the first peak of the chromatography (M). These maximum, near 1250 and 1750 cm⁻¹, denote the presence of acetylations pertaining to the native state of this polysaccharide. These bands were not observed at the peak corresponding to the total elution volume (TEV) of the column.

### Example 2:

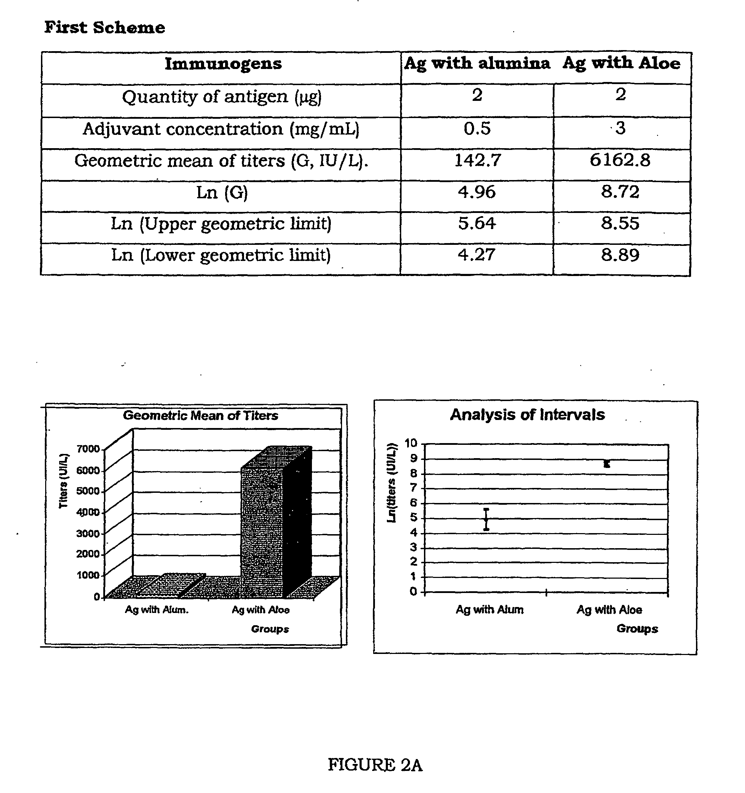

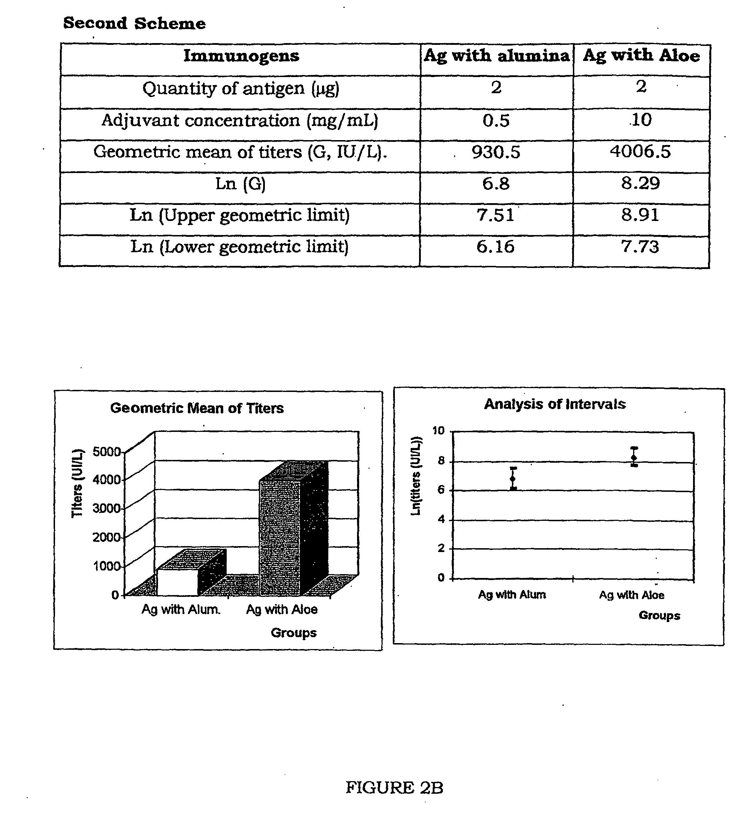

With the aim of evaluating the immunological potentiator activity of the extract from the leaf of the *Aloe barbadensis* Miller on the surface antigen of the Hepatitis B (HBsAg) virus, immunization schedules were carried out by the intraperitoneal route in male Balb/c mice of 6 to 8 weeks of age. The comparison was carried out taking as a reference the vaccine Heberbiovac-HB (produced at CIGB, Cuba) whose antigen is adsorbed in aluminum hydroxide. The immunization schedules used are specified for each case (Figures 2a and 2b).

The detection of the immune response to the HBsAg was performed by ELISA total anti-HBsAg, established for the quality control of the vaccine. The antibody titers are expressed in international units per liter. The statistical analysis of the results was performed by the Student test: p<0.05 was considered as the significant difference.

There were significant differences between both groups in the schedules. It was demonstrated with this example that it is possible to significantly potentiate the immune response to HBsAg in relation to the aluminum hydroxide after one or two intraperitoneal inoculations.

### Example 3

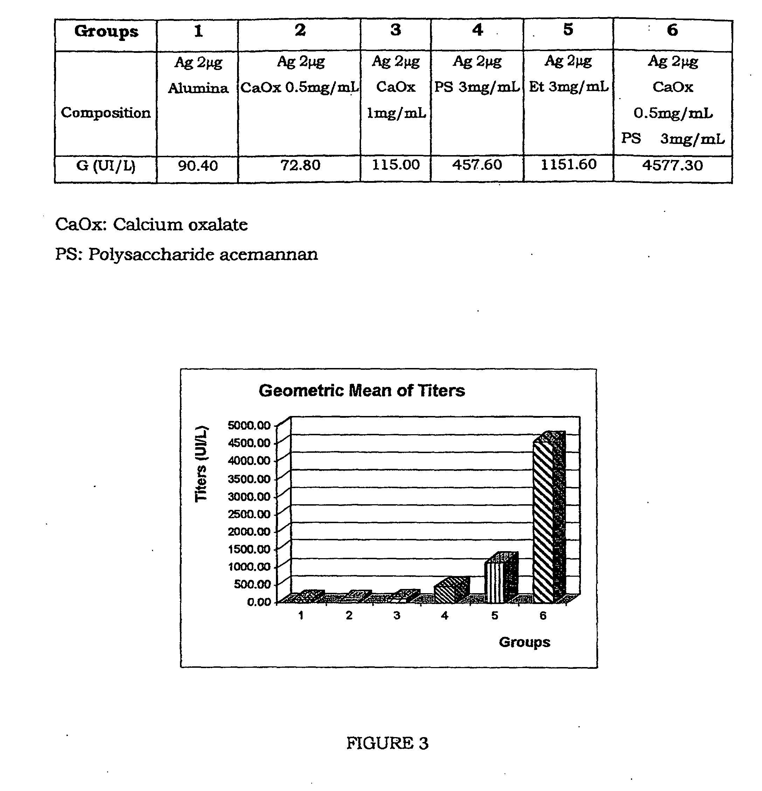

With the objective of evaluating two of the components of the extract based on its possibilities of potentiating the immune response, the acemannan and the calcium oxalate were selected. The aluminum hydroxide and the Te were used as controls. The amounts tested and the results by experimental group are shown in the table in figure 3.

This immunogenicity test was preceded by experiments on adsorption of the HBsAg to the calcium oxalate, where it was evidenced that this was possible.

The adsorption of the calcium oxalate (present in the form of a colloidal sol in the Te of the Aloe) could generate a synergic effect with the acemannan in the potentiation of the immune response.

Blood extraction was carried out after 28 days by retro-orbital piercing. The assessment of the results was performed by the ELISA anti total HBsAg, already mentioned.

Group 6 containing the combination of the acemannan and the calcium oxalate, had a significantly higher antibody response than the rest of the groups.

A synergism was evidenced between the calcium oxalate and the acemannan, with them it was possible to reconstitute and surpass the immunological potentiator activity of the total extract. In this case it is shown that the particulation of the HBsAg on the calcium oxalate sol favors the presentation of this antigen to the immunological system.

### Example 4:

To determine the immunological potentiator activity of the acemannan through the mucosal route, immunogenicity tests in Balb/c mice of 7 to 10 weeks of age were carried out using the surface antigen model of the hepatitis B virus (HBsAg). The inoculation was carried out through the nasopharyngeal route in volumes of 50 µL in anaesthetized mice. The extraction was carried out by retro-orbital piercing at 28 days after the start of the schedule. Titer determination was carried out by anti-total HBsAg ELISA. The statistical analysis was performed by the Student test: p<0.05 was considered the significant difference.

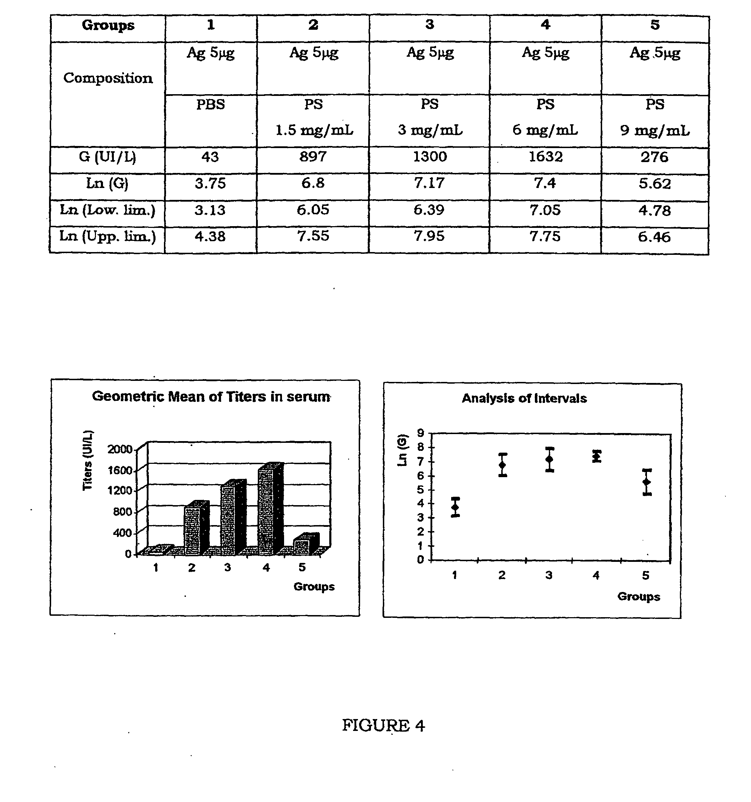

Different dose levels of the acemannan were tested, the control used was the HBsAg in PBS. The antigen was used in only one level: 5µg/dosis. Results are shown in the table of figure 4.

A strong immunological potentiator activity was evidenced in the groups in which acemannan was added. All groups were significantly superior to the control of HBsAg in PBS. The excesive increase of the generated an inhibitory effect (Group 5). This could be due to an increase of the resulting viscosity.

### Example 5

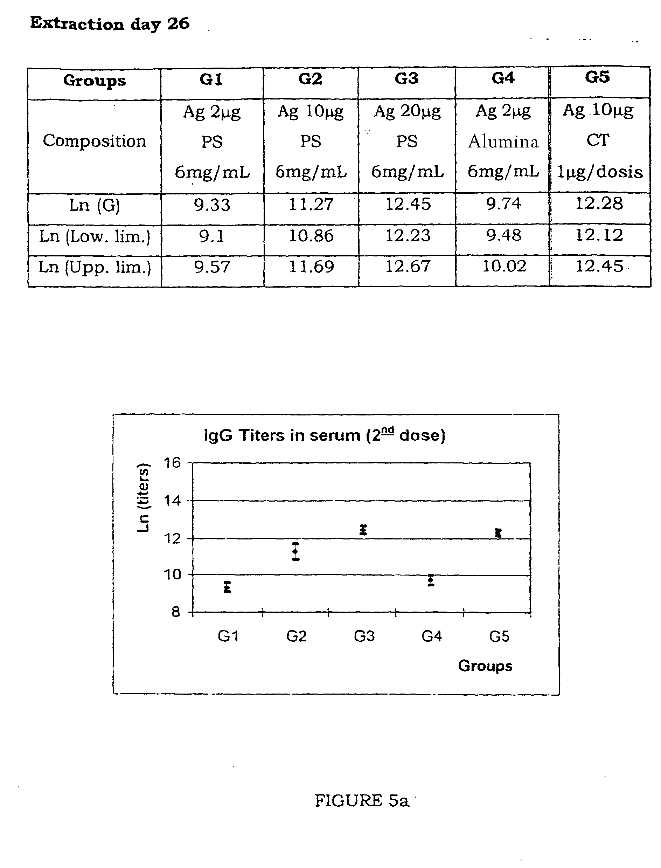

With the objective of comparing the immunological potentiator activity of acemannan for HBsAg with other reference mucosal adjuvant, an immunization schedule was carried out to compare it with a formulation of HBsAg and the cholera toxin (CT) (Figures 5a-d). Several antigen doses were assayed and the systemic inoculation of HBsAg in alumina was also used as a control. The schedule was carried out in 6-8 weeks old Balb/c female mice with four inoculations the days 0, 14, 28 and 56. Extractions were performed the days 26, 42 y 70. The evaluation of the sera was done with the conventional ELISA for the detection of specific mouse IgG antibodies. The sera were analyzed 14 days after the second, third and fourth dose.

Another objective of this assay was to compare the humoral immune response kinetics in the groups involved in the study.

An statistical analysis after a second dose did not evidenced significant differences between the alumina control group (G4) and the nasal group with the same quantity of antigen and acemannan (G1). The group of mice immunized with CT as adjuvant by the nasopharyngeal route (G5) generated an antibody response higher than that of the group with the lower quantity of antigen and was not significantly different from that of the group with equal quantity of vaccine (G2) (Figure 5a).

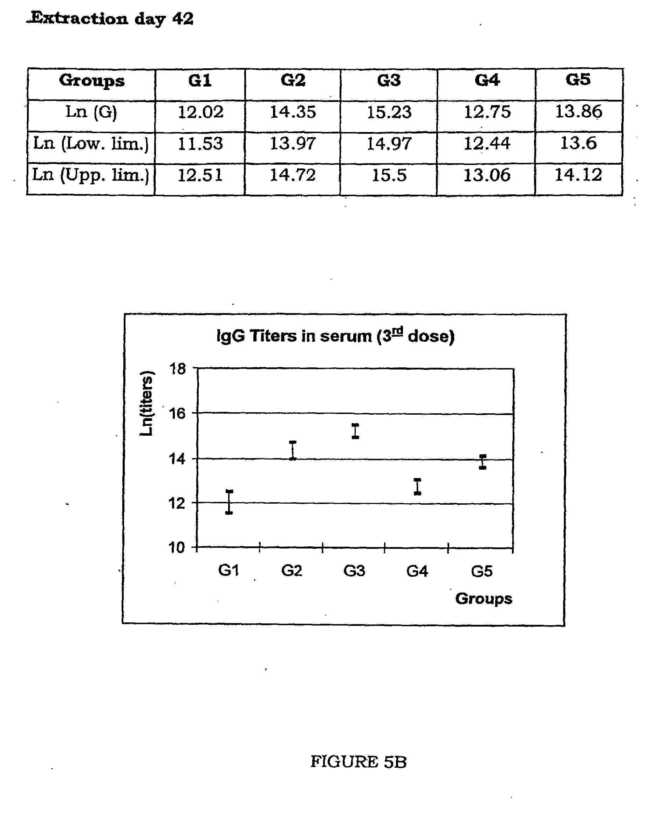

After three immunizations, there was no statistical significant difference between the group of mice immunized intranasally and systemically with 2µg (G1 and G4). In the same way, there was no significant difference between the group of mice immunized with CT as adjuvant (G5) and the equivalent formulation under assay (G2), both with con 10µg of HBsAg (Figure 5b).

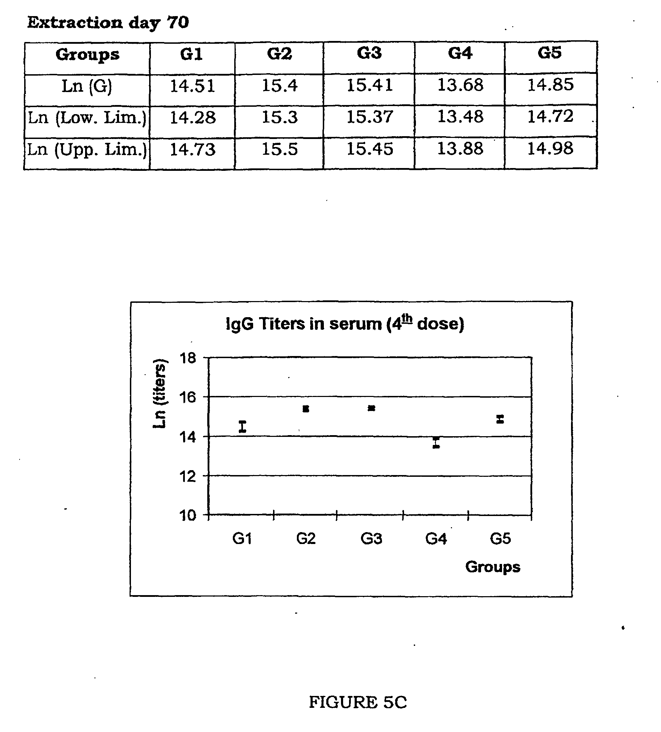

Having into account the strong increase in titers from the second to the third dose, we undertook the task of proving if this increase followed the same slope for the different groups assayed after a fourth dose, applied one month after the third inoculation. The results are shown in figure 5c.

Using acemannan as an adjuvant it is possible to obtain after a fourth dose a response which is higher than that obtained with CT in serum, as evidenced comparing G2 and G5.

The kinetics of the response shows a consistent increase, which after the third and fourth dose exceed the response generated by CT. The antibody levels generated by intranasal inoculation of 2 µg of Ag per dose (G1) were significantly higher to those obtained by the same quantity of antigen administered with alumina by systemic route (G4). This is the first evidence that it is possible to attain through the nasopharyngeal inoculation of inactive particulated antigens, adjuvanted with polysaccharides, responses which are able to exceed in quantity and quality the response generated by systemic inoculations using alumina. Additionally, the fact that a strong mucosal response was only induced by nasopharingeal route, becomes a real possibility the efficient and potentially innocuous human inoculation of HBsAg. This is an special case of particulated antigen of proteo-lyposomal nature. Other antigens may be used by this route, in order to attain a systemic response of equal strength and higher quality. From this result it may be concluded that other yeast derived antigens which get particulated generally with a size similar to HBsAg, could be used with good results by intranasal route.

### IgA quantification in vagina (day 70th)

It is of great importance to attain a mucosal response if it is considered that the sexual is one of the transmission routes of hepatitis B and other diseases.

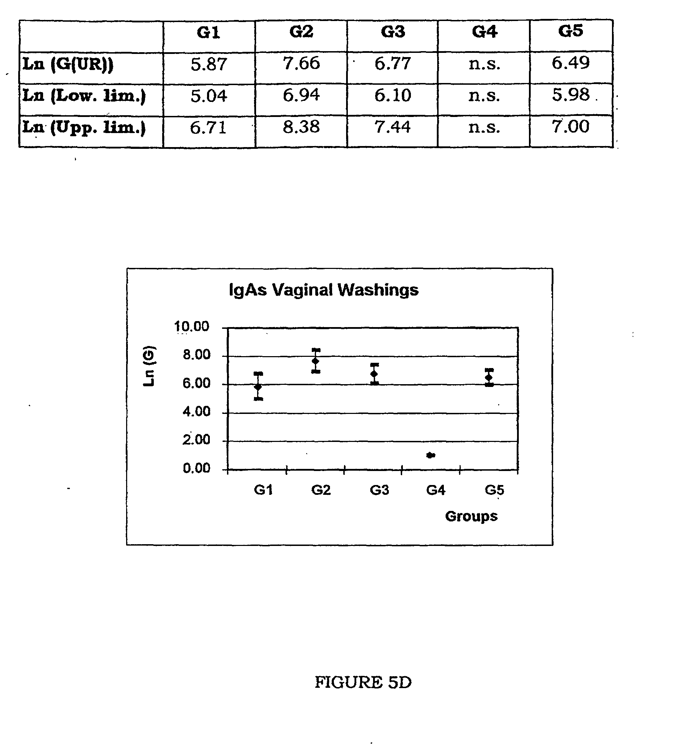

With the objective of comparing the vaginal anti-HBsAg IgA response; vaginal washings were performed with 100 of PBS after 70 days from the first inoculation and the anti-IgA antibody response was analyzed by a conventional ELISA. The results were quantified related to a vaginal washing with a high titer which served as a positive control, therefore relative units (RU) were considered (Figure 5d).

In the vaginal mucose of mice immunized with alumina as adjuvant by systemic route (group 4) an anti-HBsAg IgA response was not detected. This result supports the importance of mucosal immunization and specifically the adjuvant's potency, able to generate more strong responses in the groups 2 and 3 even though the response was not statistically significant. The antibody levels attained with 2 µg and the polysaccharide, were not statistically different to those attained with 10 µg and CT.

### Example 6

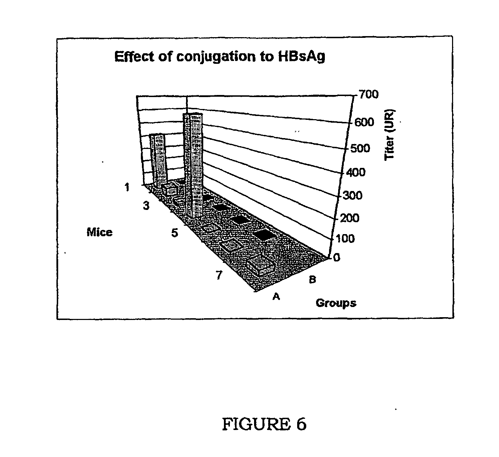

With the objective of demonstrating the possibility of delivering antigens to mucoses HBsAg was used to bind soluble antigens by chemical conjugation to a multiantigenic peptide (MAP) Th-B, formed by the universal Th epitope (830-843) of the tetanus toxin and a B peptide of the HIV gp 120 V3 loop. The intranasal inoculation was performed as previously described and the blood extraction was also done by retro-orbital puncture 42 days after the schedule was initiated. The determination of the titers was carried out by a B peptide specific anti IgG ELISA. The titers were determined related to a positive control and therefore relative units (RU) were considered (Figure 6).

In the group B there was no seroconversion. In the group A there was 100% seroconversion, the presence of two high responders mice affects the graphic visibility of the other two which seroconverted but with a lower titer.

Although the MAP (40µg) was much more represented in the group B regarding to what might be of the same in 20µg of the HBsAg-MAP conjugate (group A), there was no serum response in the group B. As seroconversion criteria twice the average values of the optical densities of negative controls were considered (mice immunized with 20µg HBsAg by the nasopharyngeal route). This result evidenced that particulation more than the presence of Th epitopes plays an important role in the response to the antigen.

As known by the physiology of the inoculation route, particulation is important, but other known mucosal adjuvants such as CT subvert this need. Therefore we may find in the literature numerous examples of immunological potentiating activity of CT for non particulated antigens, among them simple peptides. This effect does not take place with the polysaccharide but with the help of particulation.

### Exemple 7

The recombinant polypeptide of the Opc protein, one of the *Neisseria meningitidis* outer membrane proteins, was incorporated in lyposomes. Its response was evaluated in mice by the nasopharyngeal route by comparing with the response generated by the soluble polypeptide.

Initially the immunological potentiating activity on the lyposome was demonstrated. Two groups of 10 Balb/c mice were immunized. The group A was immunized with 20µg of lyposome encapsulated protein and the group B was immunized with 20µg of the same preparation adding acemannan at a final concentration of 6 mg/mL. The humoral response was evaluated a systemic level and in lung washings.

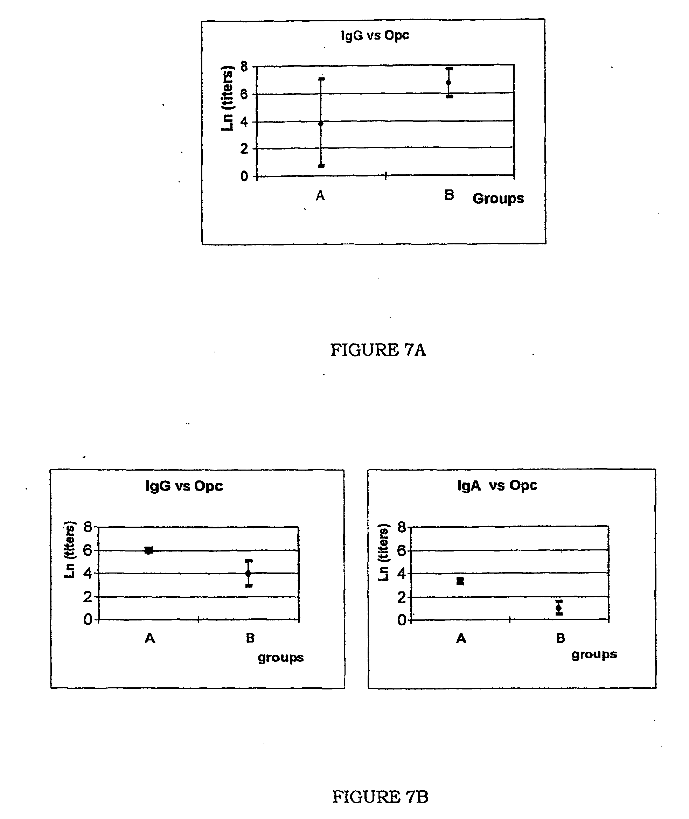

The IgG levels were measured by ELISA and were represented as logarithms of the arbitrary units assigned to each serum. The group B showed the higher levels of serum IgG, the difference was statistically significant (Figure 7a). This experiment showed the capacity of acemannan to potentiate the immune response to lyposomal formulations.

A second experiment was designed to compare the potentiating effect of the polysaccharide on the Opc protein in lyposomes (A) and on the renatured Opc protein in octyl-glucoside 10 mg/mL (B). The use of this detergent increased the recognition of the protein by four specific monoclonal antibodies for the natural Opc protein. Two of these antibodies recognize conformational epitopes and the other two lineal epitopes. According to the recognition criteria by monoclonal antibodies, the octyl-glucoside renatured Opc protein presents a conformation closer to the natural protein.

Two groups of 10 Balb/c mice were immunized intranasally. The antibody levels were quantified in the sera and the lung washings and represented as logarithms of the arbitrary units assigned to each sample (figure 7b).

In serum as well as in the mucosal washings it was observed that the combination of the lyposomes with the polysaccharide rendered the higher immunoglobulin titers and the lesser dispersion in the response. The acemannan significantly potentiates the response to the antigen when this is presented to the immune system by the lyposome (delivery system for particulated antigens).

### Example 8

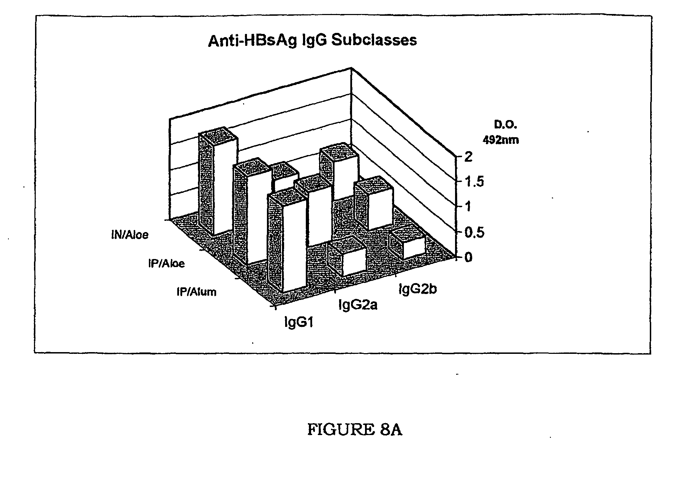

With the objective of analyzing quantitatively the response, the IgG1, 2a y 2b levels were quantified by a conventional ELISA, using specific anti IgG1, 2a y 2b conjugates. In the experiment the responses obtained with acemannan as adjuvant by IN route (IN/Aloe) were compared to the antigen inoculated by intraperitoneal route in polysaccharide and Calcium oxalate (IP/Aloe) and with the response obtained using alumina adjuvant (IP/Alum) as a control (Figure 8a).

The increase of the IgG2a antibody levels associated to the immunostimulant group under study, allows the use of this type of formulations in the field of the preventive and therapeutic vaccines requiring a strong cellular response.

According to what has been previously stated, it is possible to attain an increase of the immune response level to the surface antigen by an increase in the level of IgG2a and IgG2b.

Among the elements which may be determinant to obtain a protecting immune response are included not only those regarding to its intensity but also to its quality, i.e., the type of response which is generated as a result of the immunization. In the last years one of the topics which are more frequently dealt with in immunology is the type of helper T lymphocyte response (Th1/Th2). Many authors have demonstrated that the effective response for intracellular pathogens and viruses is the Th1 type, which correlates with the cytotoxic response and with an increase in the IgG2a subclass with respect to the other subclasses.

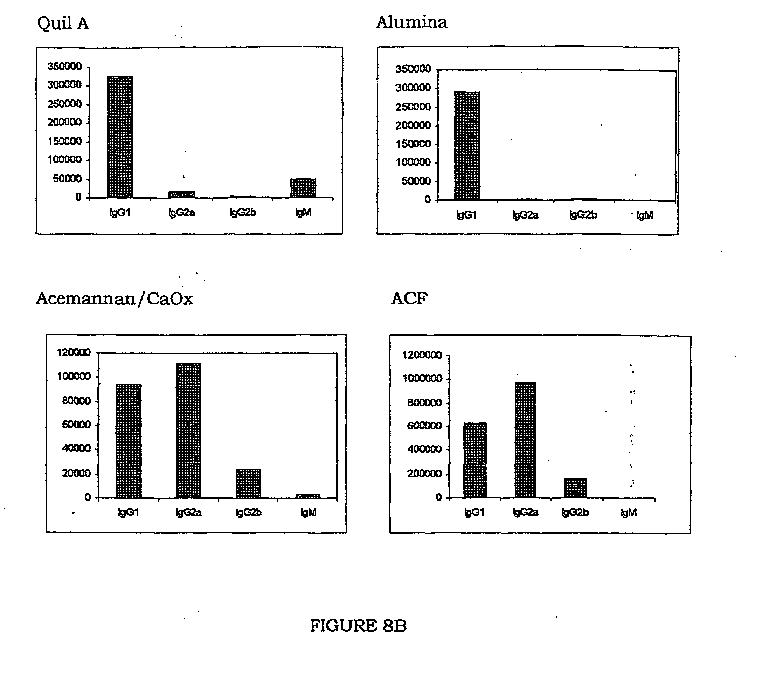

With the objective of analyzing if there exists the same performance for physically different antigens an immunization schedule was performed using a multiepitopic polypeptide (TAB9), integrated by regions of the V3 loop of the human immunodefficiency virus (HIV). The quantification of anti TAB9 immunoglobulins was done by a conventional ELISA test for the determination of IgG subclasses. 10 µg of the protein were inoculated by intraperitoneal route with different adjuvants (Figure 8b).

According to the results shown a displacement of the Th1/Th2 equilibrium is evidenced towards the Th1 response type. The level of IgG2b with de acemannan/oxalate combination is larger than the generated by using Quil A.

This result is a new piece of knowledge for the formulation as well as for the polysaccharide and allows its use not only to potentiate responses but also to modulate the responses towards Th1, which is an immunity requirement for a large number of pathogens and cancer.

### DESCRIPTION OF FIGURES

FIGURE 1. HPLC Chromatography. AD: Dextran blue (2 000 kDa), M: sample, VET: Total elution volume. Chromatographic processor BioCROM Version 2.3 (1994). Detector: Refraction index (Knauer). Gel: TSK G6000PW (Fractionation range: 500-50 000 kDa). Dimensions: 7,5 x 600 mm. Flow rate: 0,5 ml/min. Buffer: PBS. Volume of sample applied: 100 µl.
FIGURE 2a. 2 dose schedule (0, 14 days) y extraction a los 28 days.
FIGURE 2b. One dose schedule and extraction at 28 days.
FIGURE 3. One dose schedule intraperitoneally and extraction at 28 days.
FIGURE 4. 0, 14 schedule and extraction at 28 days.
FIGURE 5a. Evaluation of sera after the second dose.
FIGURE 5b. Evaluation of sera after the third dose.
FIGURE 5c. Evaluation of sera after a fourth dose.
FIGURE 5d. Evaluation of the vaginal washings (day 70)
FIGURE 6. The schedule used was 0, 14, 28 and the extraction at 42 days. A: 20 µg HBsAg conjugated to A: MAP+polysaccharide (6 mg/mL), B: 40 µg MAP+ polysaccharide (6 mg/mL).
FIGURE 7a. Three dose schedule: 0, 28, 56 and extraction at 63 days.
FIGURE 7b. Three dose schedule: 0, 28, 56 and extraction at 63 days.
FIGURE 8a. Three dose schedule 0, 14, 28 and extraction at 42 days. The sera from each variant were grouped for analytical purpose. The working dilution of samples was 1:4000.
FIGURE 8b. Three dose schedule 0, 14, 28 and extraction at 42 days. The geometric mean of titers are shown after immunization with the multiepitopic TAB9 preparation with different adjuvants.

## Claims

1. A nasopharyngeal vaccine formulation comprising a particulated antigen in soluble state and the polysaccharide acemannan as adjuvant.

2. A nasopharyngeal vaccine formulation according to claim 1, wherein the acemannan is a fraction or total extract from leaves of *Aloe barbadensis* Miller.

3. A nasopharyngeal vaccine formulation according to claim 1 or 2, wherein the acemannan concentration is in the range of 1 to 12 mg/ml.

4. A nasopharyngeal vaccine formulation according to any one of claims 1-3, wherein said particulated antigen in soluble state is HBsAg (Hepatitis B surface Antigen).

5. A nasopharyngeal vaccine formulation according to any one of claims 1-3, wherein said particulated antigen in soluble state is Hepatitis B surface Antigen chemically conjugated with to multiantigenic peptide Th-B.

6. A nasopharyngeal vaccine formulation according to any one of claims 1-3, wherein said particulated antigen in soluble state is *Neisseria meningitidis* outer membrane protein 5C incorporated in liposomes.

7. Use of acemannan for manufacturing a vaccine formulation for vaccination of a human or animal via nasopharyngeal administration, said formulation comprising a particulated antigen in soluble state and the polysaccharide acemannan as adjuvant.

8. Use of acemannan according to claim 7, wherein said vaccine formulation is for prophylactic or therapeutic treatment of a disease in a human or animal.

9. A process for manufacturing a nasopharyngeal vaccine formulation which comprises an antigen and an adjuvant, wherein a particulated antigen in soluble state is used as the antigen and the polysaccharide acemannan is used as the adjuvant.

## Patentansprüche

1. Nasenrachen-Impfstoffzubereitung, die ein teilchenförmiges Antigen in löslichem Zustand und das Polysaccharid Acemannan als Adjuvans umfasst.

2. Nasenrachen-Impfstoffzubereitung gemäß Anspruch 1, wobei es sich bei dem Acemannan um eine Fraktion oder einen Gesamtextrakt aus Blättern von *Aloe barbadensis* Miller handelt.

3. Nasenrachen-Impfstoffzubereitung gemäß Anspruch 1 oder 2, wobei die Acemannan-Konzentration im Bereich von 1 bis 12 mg/ml liegt.

4. Nasenrachen-Impfstoffzubereitung gemäß einem der Ansprüche 1 bis 3, wobei es sich bei dem teilchenförmigen Antigen in löslichem Zustand um HBsAg (Hepatitis-B-Oberflächenantigen) handelt.

5. Nasenrachen-Impfstoffzubereitung gemäß einem der Ansprüche 1 bis 3, wobei es sich bei dem teilchenförmigen Antigen in löslichem Zustand um Hepatitis-B-Oberflächenantigen handelt, das chemisch mit dem multiantigenen Peptid Th-B konjugiert ist.

6. Nasenrachen-Impfstoffzubereitung gemäß einem der Ansprüche 1 bis 3, wobei es sich bei dem teilchenförmigen Antigen in löslichem Zustand um *Neisseria-meningitidis*-Außenmembranprotein 5C handelt, das in Liposomen eingebaut ist.

7. Verwendung von Acemannan zur Herstellung einer Impfstoffzubereitung zum Impfen eines Menschen oder eines Tiers durch Nasenrachen-Verabreichung, wobei die Zubereitung ein teilchenförmiges Antigen in löslichem Zustand und das Polysaccharid Acemannan als Adjuvans umfasst.

8. Verwendung von Acemannan gemäß Anspruch 7, wobei die Impfstoffzubereitung zur prophylaktischen oder therapeutischen Behandlung einer Krankheit bei einem Menschen oder Tier dient.

9. Verfahren zur Herstellung einer Nasenrachen-Impfstoffzubereitung, die ein Antigen und ein Adjuvans umfasst, wobei ein teilchenförmiges Antigen in löslichem Zustand als Antigen und das Polysaccharid Acemannan als Adjuvans verwendet werden.

## Revendications

1. Formulation vaccinale nasopharyngée, comprenant un antigène sur particules à l'état soluble et, comme adjuvant, du polysaccharide acémannane.

2. Formulation vaccinale nasopharyngée, conforme à la revendication 1, dans laquelle l'acémannane est une fraction ou un extrait total tiré de feuilles d'*Aloe barbadensis* Miller.

3. Formulation vaccinale nasopharyngée, conforme à la revendication 1 ou 2, dans laquelle la concentration d'acémannane se situe dans l'intervalle allant de 1 à 12 mg/ml.

4. Formulation vaccinale nasopharyngée, conforme à l'une des revendications 1 à 3, dans laquelle ledit antigène sur particules à l'état soluble est de l'antigène de surface du virus de l'hépatite B, HBsAg.

5. Formulation vaccinale nasopharyngée, conforme à l'une des revendications 1 à 3, dans laquelle ledit antigène sur particules à l'état soluble est de l'antigène de surface du virus de l'hépatite B, chimiquement conjugué au peptide multiantigène Th-B.

6. Formulation vaccinale nasopharyngée, conforme à l'une des revendications 1 à 3, dans laquelle ledit antigène sur particules à l'état soluble est de la protéine 5C de membrane externe de *Neisseria meningitidis,* incorporée dans des liposomes.

7. Emploi d'acémannane pour la fabrication d'une formulation vaccinale destinée à la vaccination d'un humain ou d'un animal par administration nasopharyngée, ladite formulation comprenant un antigène sur particules à l'état soluble et, comme adjuvant, du polysaccharide acémannane.

8. Emploi d'acémannane selon la revendication 7, ladite formulation vaccinale étant destinée au traitement prophylactique ou thérapeutique d'une maladie chez un humain ou un animal.

9. Procédé de fabrication d'une formulation vaccinale nasopharyngée, comprenant un antigène et un adjuvant, dans lequel on emploie comme antigène un antigène sur particules à l'état soluble, et comme adjuvant, du polysaccharide acémannane.
